# EUROPEAN PATENT SPECIFICATION

(11) **EP 3 457 123 B1**
(45) Date of publication and mention of the grant of the patent: **21.09.2022**
(21) Application number: 16891652.6
(22) Date of filing: 09.12.2016
(51) Int. Cl.: G01N 33/50

(54) **METHOD FOR ANALYZING SIALYL SUGAR CHAIN**
VERFAHREN ZUR ANALYSE DER SIALYLZUCKERKETTE
PROCÉDÉ D'ANALYSE DE CHAÎNE DE SUCRE DE TYPE SIALYLE

(30) Priority: 22.02.2016 JP 2016030603
(43) Date of publication of application: 20.03.2019
(73) Proprietor: Shimadzu Corporation, Kyoto-shi, Kyoto 604-8511 (JP)
(72) Inventor: NISHIKAZE, Takashi, Kyoto-shi Kyoto 604-8511 (JP)
(74) Representative: Mewburn Ellis LLP
(86) International application number: PCT/JP2016/086764
(87) International publication number: WO 2017/145496

(56) References cited:
- JP-A- 2008 151 514
- JP-A- 2010 249 576
- JP-A- 2014 115 087
- JP-A- 2015 034 712
- JP-A- 2015 158 510
- K R REIDING ET AL: "High-Throughput Profiling of Protein N-Glycosylation by MALDI-TOF-MS Employing Linkage-Specific Sialic Acid Esterification", ANALYTICAL CHEMISTRY, vol. 86, no. 12, 17 June 2014 (2014-06-17) , pages 5784-5793, XP055316180, US DOI: 10.1021/ac500335t
- M R BLADERGROEN ET AL: "Automation of High-Throughput Mass Spectrometry-Based Plasma N-Glycome Analysis with Linkage-Specific Sialic Acid Esterification", JOURNAL OF PROTEOME RESEARCH, vol. 14, no. 9, 4 September 2015 (2015-09-04), pages 4080-4086, XP055661384, DOI: 10.1021/acs.jproteome.5b00538
- N DE HAAN ET AL: "Linkage-Specific Sialic Acid Derivatization for MALDI-TOF-MS Profiling of IgG Glycopeptides", ANALYTICAL CHEMISTRY, vol. 87, no. 16, 31 July 2015 (2015-07-31) , pages 8284-8291, XP055460535, US DOI: 10.1021/acs.analchem.5b02426 -& N De Haan et al: "Supporting information: Linkage-specific sialic acid derivatization for MALDI-TOF-MS profiling of IgG glycopeptides Correspondence", Analytical Chemistry, 20 July 2015 (2015-07-20), XP055661451, Retrieved from the Internet: URL:https://pubs.acs.org/doi/suppl/10.1021 /acs.analchem.5b02426/suppl_file/ac5b02426 _si_001.pdf [retrieved on 2020-01-23]
- T NISHIKAZE ET AL: "Differentiation of Sialyl Linkage Isomers by One-Pot Sialic Acid Derivatization for Mass Spectrometry-Based Glycan Profiling", ANALYTICAL CHEMISTRY, vol. 89, no. 4, 8 February 2017 (2017-02-08), pages 2353-2360, XP055660957, DOI: 10.1021/acs.analchem.6b04150
- TAKASHI NISHIKAZE et al.: "Negative-ion MALDI-MS2 for discrimination of 2,3- and 2,6-sialylation on glycopeptides labeled with a pyrene derivative.", Journal of Chromatography B, vol. 879, no. 17-18, May 2011 (2011-05), pages 1419-1428, XP028203532,

## Description

### TECHNICAL FIELD

The present invention relates to a method using mass spectrometry for analyzing the structure of a glycan having a sialic acid at a terminal, and more specifically, to an analysis method for analyzing the structure of a sialylated glycan taking into account a distinction between different linkage types of the sialic acid.

### BACKGROUND ART

It is commonly said that more than one half of the proteins forming living organisms are have their glycans modified. Glycan modification plays an important role for the adjustment of the structure and function of proteins. Meanwhile, recent studies have revealed that glycans expressed on the cell surface contribute to various biological phenomena, such as the cellular interaction, signaling, development/differentiation, fertilization, or cancer metastasis. It has also been known that monosaccharides, such as sialic acids, are linked to the non-reducing terminals of glycans. Monosaccharides linked to such terminals have been found to play important roles for the function of glycans.

Sialic acid is a generic term for compounds formed by a substitution of an amino group or hydroxy group in a neuraminic acid which is a special kind of nine-carbon sugar to which an amino group and a carboxy group are linked. N-acetylneuraminic acid (Neu5Ac), in which the amino group at the C5 position is acetylated, is considered to be the most abundant in nature. There are also various other known structures, such as N-glycolylneuraminic acid (Neu5Gc) in which the amino group at the fifth position is modified with a glycolyl group, and KDN, which is a deaminated neuraminic acid.

Needless to say, the number of sialic acids linked to the terminals of a glycan is important in terms of the previously mentioned functions of the glycan in living organisms. Additionally, the linkage type, i.e. the way in which the sialic acid residue is attached, is also important. For example, α2,3-linkage and α2,6-linkage have been known as major linkage types for sialic acid residues in human bodies. These types are also called linkage isomers. It has been pointed out that their linkage types change with cancerization. Thus, they have been expected to be used as biomarkers. For the quality control of biological drugs or other purposes, it is necessary to ensure not only the consistency of the amino acid sequence in glycoproteins or glycopeptides but also the control of their structures after a posttranslational modification. Thus, it has been demanded to establish an analyzing technique for the control of the glycan modification including the linkage type of the sialic acid residue, as well as one for the quality control.

For an analysis of the linkage type of a sialic acid residue in a glycan, several techniques have been proposed in which a chemical modification that is specific to the linkage type is performed. Those techniques primarily utilize the fact that the α2,3-sialic acid residue more easily undergoes intramolecular dehydration by a dehydration condensation agent than the α2,6-sialic acid residue. For example, those proposed techniques convert a glycan into lactone and methyl ester by a reaction with methanol (see Non-Patent Literature 1), into lactone and ethyl ester by a reaction with ethanol (see Non-Patent Literature 2 and 9), into lactone and amide by a reaction with ammonium chloride (see Non-Patent Literature 3), or into lactone and dimethyl amide by a reaction with dimethyl amine (see Non-Patent Literature 4). Additionally, the present inventor and associates have demonstrated that the α2,3-sialic acid residue and α2,6-sialic acid residue can be distinguished from each other with a high level of accuracy through amidation using isopropyl amine, as disclosed in Non-Patent Literature 5.

For a structural analysis of a glycan using mass spectrometry, it is normally necessary to initially estimate the composition of the monosaccharides forming a glycan to be analyzed, based on the mass-to-charge-ratio values obtained by a mass spectrometric analysis performed on the glycan. For this estimation, the kinds of monosaccharides potentially present in the glycan and the range of the number of occurrences of each monosaccharide are specified as search conditions in a data analyzer. Analytical parameters are also set, such as the permissible mass accuracy. Then, an exhaustive searching process is carried out. This is a simple process: the accurate masses (which are previously known) and numbers of monosaccharides are appropriately combined to search for a combination that is consistent with the measured mass-to-charge-ratio values. Through this process, one or more computationally possible compositions of monosaccharides are presented as monosaccharide composition candidates. The conditions of this search may include a setting for distinguishing between linkage-type-specific sialic acid residues, or more specifically, between the modification of the α2,3-sialic acid residue and that of the α2,6-sialic acid residue. Setting such a condition dramatically increases the number of eventually obtained monosaccharide composition candidates, as compared to the case where no distinction is made between the linkage types. This occurs due to the fact the number of monosaccharide residues corresponding to the sialic acid simply becomes more than double. Such an increase in the number of monosaccharide composition candidates makes it difficult for analysis operators to narrow down the candidates, which increases the period of time for the task as well as lowers its accuracy.

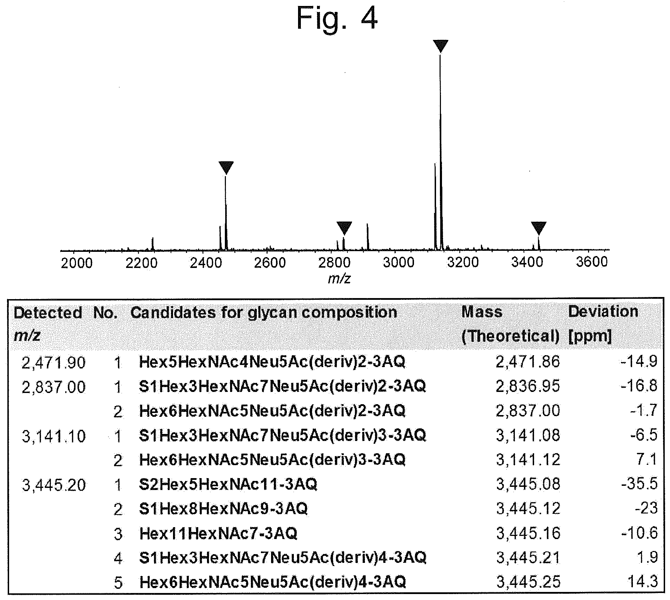

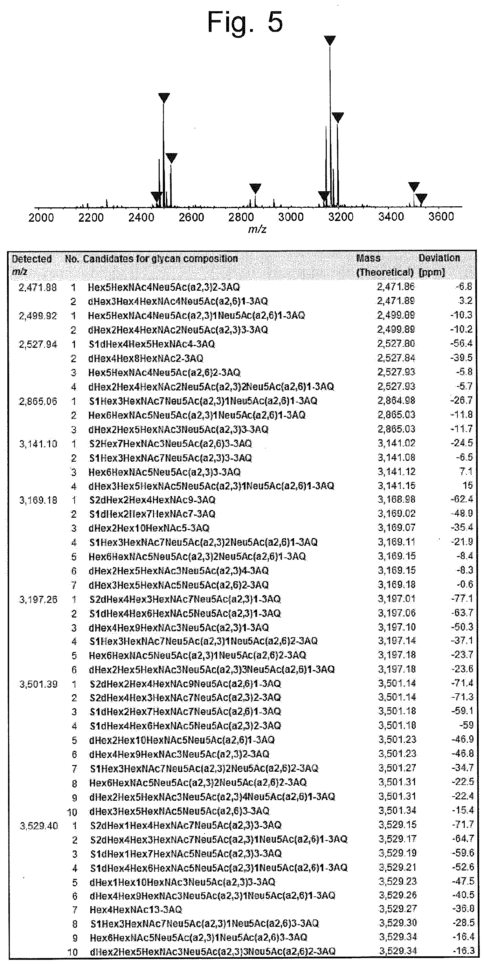

One example is hereinafter described with reference to Figs. 4 and 5. Each of Figs. 4 and 5 shows a measured mass spectrum and the result of a monosaccharide composition search performed on the major peaks on the mass spectrum.

In the case of an analysis of an N-linked glycan derived from bovine fetuin, if a mass spectrometric analysis over a predetermined range of mass-to-charge ratios is performed on a sample in which sialic acid residues have been modified in a way that is non-specific to the linkage type, four major peaks as indicated by the inverted black triangles in Fig. 4 will be observed on the mass spectrum. If a monosaccharide composition search is performed under predetermined conditions based on this mass spectrometric result, the total number of monosaccharide composition candidates to be eventually obtained will not be much greater than 10. By comparison, if a similar mass spectrometric analysis is performed on a sample in which sialic acid residues have been modified in a linkage-type-specific way, the number of major peaks observed on the mass spectrum increases to nine, as shown in Fig. 5. If a monosaccharide composition search is performed based on this mass spectrometric result under the same conditions as mentioned earlier, the total number of monosaccharide composition candidates to be eventually obtained increases to 48. The extent of the increase in the number of monosaccharide composition candidates is far greater than that of the increase in the number of observed peaks. The task of individually checking such a large number of candidates to select appropriate candidates will be extremely cumbersome for the analysis operator.

In the aforementioned calculation, only Neu5Ac is taken into account as the sialic acid. However, other than Neu5Ac whose nominal mass is 291, Neu5Gc having a nominal mass of 307 is also commonly known as a sialic acid residue in sialylated glycans. It is preferable to consider at least these two kinds of sialic acid residues in a monosaccharide composition search. This further increases the number of masses of the modified sialic acid residues generated by a non-linkage-specific modification, and the number of monosaccharide compositions to be obtained by the search will also be extremely large.

### CITATION LIST

### NON PATENT LITERATURE

Non-Patent Literature 1: Wheeler SF and two other authors, "Derivatization of sialic acids for stabilization in matrix-assisted laser desorption/ionization mass spectrometry and concomitant differentiation of alpha(2 --> 3)- and alpha(2 --> 6)-isomers", Rapid Commun. Mass Spectrom., January 2009, Vol.23, No.2, pp.303-312
Non-Patent Literature 2: K. R. Reiding and four other authors, "High-Throughput Profiling of Protein N-Glycosylation by MALDI-TOF-MS Employing Linkage-Specific Sialic Acid Esterification", Anal. Chem., 2014, Vol.86, No.12, pp.5784-5793
Non-Patent Literature 3: W. R. Alley and another author, "Glycomic Analysis of Sialic Acid Linkages in Glycans Derived from Blood Serum Glycoproteins", J. Proteome Res., June 4, 2010, Vol.9, No.6, pp.3062-3072
Non-Patent Literature 4: N. de Haan and five other authors, "Linkage-Specific Sialic Acid Derivatization for MALDI-TOF-MS Profiling of IgG Glycopeptides", Anal. Chem., 2015, Vol.87, No.16, pp.8284-8291
Non-Patent Literature 5: T. Nishikaze and two other authors, "Towards the discrimination of sialyl linkages in glycopeptides: A new derivatization approach", 63rd ASMS Proceedings, May 2015, MP-674
Non-Patent Literature 6: "Glyco-Peakfinder", [online], [accessed on January 19, 2016], the Internet <URL: http://www.glyco-peakfinder.org/>
Non-Patent Literature 7: K. Kaneshiro and four other authors, "Highly Sensitive MALDI Analyses of Glycans by a New Aminoquinoline-Labeling Method Using 3-Aminoquinoline/α-Cyano-4-hydroxycinnamic Acid Liquid Matrix", Anal. Chem., 2011, Vol.83, No.10, pp.3663-3667
Non-Patent Literature 8: Y. Fukuyama and eight other authors, "3-Aminoquinoline/p-Coumaric Acid as a MALDI Matrix for Glycopeptides, Carbohydrates, and Phosphopeptides", Anal. Chem., 2014, Vol.86, No.4, pp.1937-1942
Non-Patent Literature 9: M. R. Bladergroen and eight other authors, "Automation of High-Throughput Mass Spectrometry-Based Plasma N-Glycome Analysis with Linkage-Specific Sialic Acid Esterification", J. Proteome Res., July 16, 2015, Vol.14, No.9, pp.4080-4086

### SUMMARY OF INVENTION

### TECHNICAL PROBLEM

The present invention has been developed to solve the previously described problem. Its objective is to provide a sialylated glycan analysis method capable of efficiently and accurately performing a structural analysis including a variation in the linkage type of a sialic acid residue in a sialylated glycan, by effectively narrowing down the monosaccharide composition candidates of the sialylated glycan estimated from the result of a mass spectrometric analysis.

### SOLUTION TO PROBLEM

The present invention developed for solving the previously described problem is a sialylated glycan analysis method using mass spectrometry for analyzing the structure of a sialylated glycan to which a sialic acid is linked, the method including:
a) a modification step in which a linkage-type-specific modification is performed on a sialic acid residue included in a sialylated glycan to be analyzed;
b) an analysis execution step in which the sialylated glycan after the modification by the modification step is subjected to a mass spectrometric analysis to obtain mass spectrum information;
c) a candidate estimation step in which a candidate of a monosaccharide composition is estimated for a peak observed on the mass spectrum obtained in the analysis execution step, based on mass information of the peak; and
d) a candidate-narrowing step in which a plurality of peaks observed on the mass spectrum and having a mass difference corresponding to the technique of the linkage-type-specific modification are assumed to be a cluster of peaks corresponding to sialylated glycans including sialic acid residues of the same kind with different linkage types and being identical in the monosaccharide composition exclusive of the sialic acid residues, and monosaccharide composition candidates obtained for those peaks are narrowed down by judging each monosaccharide composition candidate from at least one of the following aspects:
   (i) excluding a monosaccharide composition candidate which does not include a sialic acid residue;
   (ii) excluding a monosaccharide composition candidate which does not include a specific type of modified sialic acid residue;
   (iii) excluding a monosaccharide composition candidate which does not include a predetermined number of a specific type of modified sialic acid residue;
   (iv) excluding a monosaccharide composition candidate which is not identical in a monosaccharide composition exclusive of the sialic acid residue.

Commonly known linkage types of sialic acid residues include α2,3-, α2,6-, α2,8- and so on. The present invention is compatible with any linkage type as long as the linkage type allow for a linkage-type-specific modification (derivatization), or in other words, as long as the modification technique allows for a distinction between different linkage types. Typically, the sialyl glycan analysis method according to the present invention is useful for a glycan structure analysis in which α2,3-sialic acid residue and α2,6-sialic acid residue should be distinguished from each other, both of which are major linkage types.

In this case, for example, an isopropyl amide modification and a methyl amide modification may be performed as the linkage-type-specific modification in the modification step. α2,6-Sialic acid residue undergoes the isopropyl amide modification, while α2,3-sialic acid residue undergoes the methyl amide modification. The two modifications have an nominal mass difference of 28 Da. A lactone modification may be used in place of the methyl amide modification, in which case the two modifications have an nominal mass difference of 59 Da. In the case of the linkage-type-specific modification method described in Non-Patent Literature 1 (lactone modification and methyl ester modification), the nominal mass difference between the two modifications is 32 Da. In the case of the linkage-type-specific modification method described in Non-Patent Literature 2 (lactone modification and ethyl ester modification), the nominal mass difference between the two modifications is 48 Da. In the case of the linkage-type-specific modification method described in Non-Patent Literature 3 (lactone modification and amide modification, followed by complete methylation), the nominal mass difference between the two modifications is 13 Da. In the case of the linkage-type-specific modification method described in Non-Patent Literature 4 (lactone modification and dimethyl amide modification), the integer mass difference between the two modifications is 45 Da.

For example, Neu5Ac, which is one of the sialic acids, has a mass of 291. Modifying α2,6-sialic acid residue with isopropyl amide and α2,3-sialic acid residue with methyl amide divides Neu5Ac into two masses; i.e. Neu5Ac modified with isopropyl amide has a mass of 332, while Neu5Ac modified with methyl amide has a mass of 304. Now, consider a glycan which includes three sialic acid residues. There are four possible combinations of the linkage types for the three sialic acid residues: All of them are α2,3- [α2,3-, α2,3-, α2,3-]; two of them are α2,3- [α2,3-, α2,3-, α2,6-]; one of them is α2,3- [α2,3-, α2,6-, α2,6-]; and all of them are α2,6- [α2,6-, α2,6-, α2,6-]. The mass of the glycan changes depending on the combination. Accordingly, a maximum of four peaks may possibly be observed on a mass spectrum at intervals of 28 Da which corresponds to the mass difference between Neu5Ac modified with isopropyl amide and Neu5Ac modified with methyl amide.

Accordingly, in the candidate-narrowing step in the present invention, a plurality of peaks having a mass difference corresponding to the technique used for the linkage-type-specific modification, e.g. 28 Da, are extracted, and those peaks are assumed to be a cluster of peaks corresponding to sialyl glycans which include a sialic acid residue with different linkage types and are identical in the monosaccharide composition exclusive of the sialic acid residues (such a cluster of peaks is hereinafter called the "isomer cluster peak"). A glycan corresponding to an isomer cluster peak must include a sialic acid. Therefore, for example, if no sialic acid residue is present in a monosaccharide composition candidate obtained for a peak which belongs to the isomer cluster peak, it is possible to determine that the candidate is not a proper candidate.

Glycans which respectively correspond to the peaks belonging to one isomer cluster peak must be identical in the monosaccharide composition exclusive of the sialic acid residue and the modified sialic acid residue. Accordingly, it is also possible to narrow down monosaccharide composition candidates by judging their identity of the monosaccharide composition exclusive of the sialic acid residue and the modified sialic acid residue.

Furthermore, for example, if there are two peaks having a mass difference of 28 Da or 56 Da, which equals two times 28 Da, the glycan corresponding to the peak having the larger mass must include the isopropyl amide modification which has a larger mass, while the glycan corresponding to the peak having the smaller mass must include the methyl amide modification which has a smaller mass. This fact can also be utilized; i.e., it is possible to narrow down monosaccharide composition candidates based on the presence or absence of a specific type of modified sialic acid residue, or number of occurrences of the specific type of modified sialic acid residue.

Thus, in the candidate-narrowing step in one mode of the present invention, for a sialyl glycan having a sialic acid linked to one or more terminals of the glycan, a plurality of peaks corresponding to the mass difference (e.g. 28 Da) between different types of modified sialic acid residue generated by the linkage-type-specific modification are located, and the monosaccharide composition candidates are narrowed down under the condition that the monosaccharide composition corresponding to a peak on the lower-mass side among the plurality of peaks should include at least one modified sialic acid residue having a smaller mass (e.g. a sialic acid residue modified with methyl amide) while the monosaccharide composition corresponding to a peak on the higher-mass side should include at least one modified sialic acid residue having a larger mass (e.g. a sialic acid residue modified with isopropyl amide).

In a more specific mode of the previously described candidate-narrowing step, two peaks corresponding to N times the mass difference ΔM between the different types of modified sialic acid residue generated by the linkage-type-specific modification (where N is an integer equal to or greater than one) are located, and the monosaccharide composition candidates are narrowed down under the condition that the monosaccharide composition corresponding to the peak on the lower-mass side should include the modified sialic acid residue having the smaller mass and occurring at N locations. For example, the modified sialic acid residue having the smaller mass and occurring at N locations is a modified α2,3-sialic acid residue.

In another specific mode of the previously described candidate-narrowing step, two peaks corresponding to N times the mass difference ΔM between the different types of modified sialic acid residue generated by the linkage-type-specific modification (where N is an integer equal to or greater than one) are located, and the monosaccharide composition candidates are narrowed down under the condition that the monosaccharide composition corresponding to the peak on the higher-mass side should include the modified sialic acid residue having the larger mass and occurring at N locations. For example, the modified sialic acid residue having the larger mass and occurring at N locations is a modified α2,6-sialic acid residue.

Theoretically, if there are three or more peaks belonging to one isomer cluster peak, it is not guaranteed that all peaks are observed. For example, in the case of a glycan which includes three sialic acid residues as described earlier, although four peaks may possibly be observed, it is often the case that only two or three peaks are actually observed (due to the other peaks being too low to be observed). Therefore, it is useful to locate not only a peak corresponding to the mass difference between different modifications but also a peak corresponding to the mass difference multiplied by two, three or more.

In the sialyl glycan analysis method according to the present invention, the estimation of a monosaccharide composition candidate in the candidate estimation step may be performed by computing the combination of monosaccharides which are consistent with the masses of the peaks under search conditions which specify the kinds of potentially contained monosaccharides and the range of the number of occurrences of each monosaccharide.

All monosaccharide composition candidates can be exhaustively extracted by this method, provided that the kinds of monosaccharides and the range of the number of occurrences of each monosaccharide are appropriately set.

In the sialyl glycan analysis method according to the present invention, the mass spectrometric analysis in the analysis execution step may preferably be performed in the negative-ion mode. As compared to a mass spectrometric analysis in the positive-ion mode, the mass spectrometric analysis in the negative-ion mode produces glycan ions in a stable form that barely undergoes unnecessary fragmentations. This is convenient for a structural analysis based on a mass spectrum. Furthermore, the negative-ion mode hardly generates adduct ions having sodium or potassium added. Therefore, noise peaks due to the adduct ions do not appear in the mass spectrum, so that the peaks originating from the target glycan can be easily extracted.

The processing in the candidate estimation step and the candidate-narrowing step in the sialyl glycan analysis method according to the present invention may be performed by executing, on a personal computer or more sophisticated computer, a piece of software (computer program) previously installed on the same computer.

### ADVANTAGEOUS EFFECTS OF INVENTION

With the sialyl glycan analysis method according to the present invention, the number of monosaccharide composition candidates of a sialyl glycan estimated from the result of a mass spectrometric analysis can be reduced by effectively narrowing down the candidates by taking into account a distinction between different linkage types of a sialic acid residue. Thus, a structural analysis including a variation in the linkage type of a sialic acid residue in a sialylated glycan can be efficiently and accurately performed.

### BRIEF DESCRIPTION OF DRAWINGS

Fig. 1 is a flow chart schematically showing an analytical procedure in a sialylated glycan analysis method as one embodiment of the present invention.
Fig. 2 is a diagram explaining a method for narrowing down monosaccharide composition candidates in the sialyl glycan analysis method according to the present embodiment (in the case where the number of sialic acid residues is two).
Fig. 3 is a diagram explaining a method for narrowing down monosaccharide composition candidates in the sialyl glycan analysis method according to the present embodiment (in the case where the number of sialic acid residues is three).
Fig. 4 is an illustration showing a measured mass spectrum for an N-linked glycan derived from bovine fetuin modified in a way that is non-specific to the linkage type of the sialic acid, as well as the result of a search for monosaccharide compositions for four major peaks.
Fig. 5 is an illustration showing a measured mass spectrum for an N-linked glycan derived from bovine fetuin modified in a way that is specific to the linkage type of the sialic acid, as well as the result of a search for monosaccharide compositions for nine major peaks.
Fig. 6 is an enlarged view of a section around m/z=2500 in a measured mass spectrum for an N-linked glycan derived from bovine fetuin modified in a way that is specific to the linkage type of the sialic acid, as well as the result of a process in which the result of the search for the monosaccharide compositions for the peaks detected within the enlarged section has been narrowed down by the method according to the present embodiment.
Fig. 7 is an enlarged view of a section around m/z=2800 in a measured mass spectrum for an N-linked glycan derived from bovine fetuin modified in a way that is specific to the linkage type of the sialic acid, as well as the result of a process in which the result of the search for the monosaccharide compositions for the peaks detected within the enlarged section has been narrowed down by the method according to the present embodiment.
Fig. 8 is an enlarged view of a section around m/z=3150 in a measured mass spectrum for an N-linked glycan derived from bovine fetuin modified in a way that is specific to the linkage type of the sialic acid, as well as the result of a process in which the result of the search for the monosaccharide compositions for the peaks detected within the enlarged section has been narrowed down by the method according to the present embodiment.
Fig. 9 is an enlarged view of a section around m/z=3500 in a measured mass spectrum for an N-linked glycan derived from bovine fetuin modified in a way that is specific to the linkage type of the sialic acid, as well as the result of a process in which the result of the search for the monosaccharide compositions for the peaks detected within the enlarged section has been narrowed down by the method according to the present embodiment.

### DESCRIPTION OF EMBODIMENTS

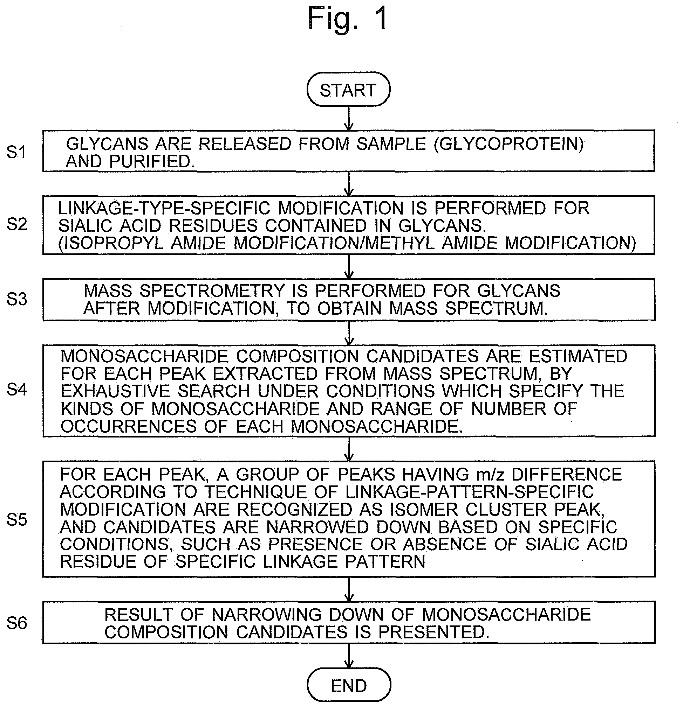

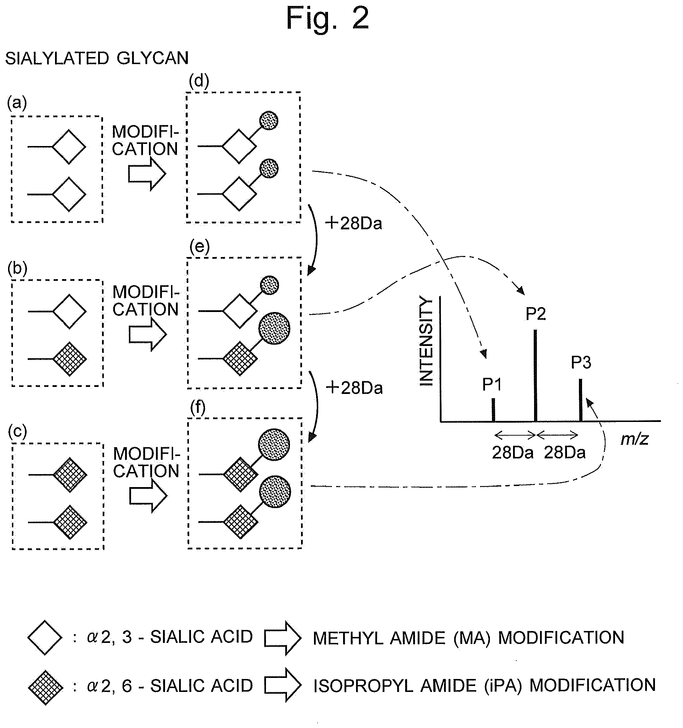

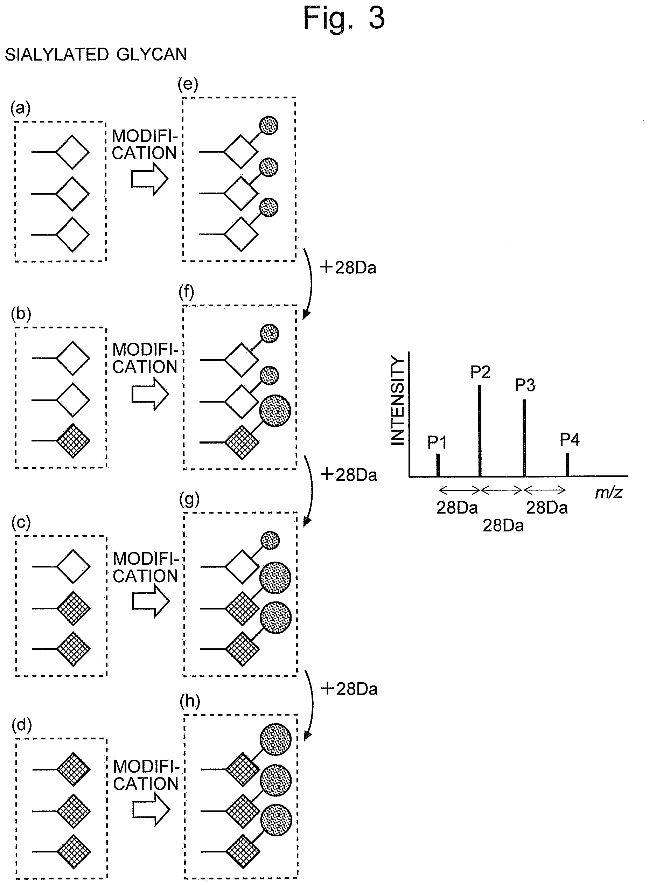

A sialylated glycan analysis method according to one embodiment of the present invention is hereinafter described in detail with reference to the attached drawings. Fig. 1 is a flow chart schematically showing an analytical procedure in the sialylated glycan analysis method according to the present embodiment. Figs. 2 and 3 are conceptual diagrams for explaining a method for narrowing down the monosaccharide composition candidates in the sialyl glycan analysis method according to the present embodiment.

Referring to Figs. 1-3, the following description initially provides an overview of the sialylated glycan analysis method according to the present embodiment as well as the characteristic method for narrowing down monosaccharide composition candidates.

Initially, a sample is prepared from a glycoprotein including glycans to be analyzed (sialylated glycans) by cutting out the glycans from the glycoprotein by a known method and then purifying the cut glycans (Step S1).

Subsequently, the sialic acids linked to the glycans contained in the prepared sample are modified by a linkage-type-specific derivatization (Step S2). Two particularly important kinds of linkage types, i.e. α2,3-linkage and α2,6-linkage, are considered in the present embodiment, although there are also other known forms for sialic acids to be linked to glycans, such as the α2,8-linkage. As noted earlier, there are several commonly known modification methods for distinguishing between the α2,3-sialic acid residue and α2,6-sialic acid residue (for example, see Non-Patent Literature 1-5). Any modification (derivatization) method may be used as long as it can modify sialic acid residues in a linkage-type-specific way. The combination of isopropyl amide modification and methyl amide modification disclosed in Non-Patent Literature 5 is used in the present embodiment. In this case, the α2,6-sialic acid residue turns into an isopropyl amide modification, while the α2,3-sialic acid residue turns into a methyl amide modification. In the following description, the sialic acid modified with isopropyl amide is called the iPA modification, while the sialic acid modified with methyl amide is called the MA modification.

As noted earlier, there are sialic acids other than Neu5Ac, such as Neu5Gc and KDN. In the present embodiment, Neu5Ac is considered, which is the most abundant. The masses to be mentioned in the following description will basically be expressed in nominal mass (an integer mass of an isotope having the highest natural abundance ratio) in order to avoid complication of the description. The mass of Neu5Ac is 291, while those of the iPA and MA modifications are 332 and 304, respectively. The two modifications have a mass difference of 28 Da.

After the sialic acid residues included in the glycans in the sample have been modified in the linkage-type-specific way as described earlier, the sample is subjected to a mass spectrometric analysis to obtain a mass spectrum covering a predetermined range of mass-to-charge ratios (Step S3). The mass accuracy should preferably be high so that the monosaccharide composition can be estimated based on mass information as will be described later. Accordingly, it is preferable to use a mass spectrometer capable of an analysis with high accuracy and high sensitivity, such as a time-of-flight mass spectrometer or Fourier transform mass spectrometer.

After the mass spectrum has been obtained, the peaks observed on the mass spectrum are extracted. For each extracted peak, the monosaccharide composition is estimated from the mass value of the peak. The estimation of the monosaccharide composition is specifically achieved by an exhaustive search for all possible combinations of monosaccharides which are consistent with the mass value of the measured peak (or practically, which fall within a predetermined numerical range) under the search conditions specified beforehand by an analysis operator, including the kinds of monosaccharaides and the range of the number of occurrences of each monosaccharide, with the permissible mass error and other items of information as analytical parameters (Step S4). Such a search can be performed on a computer, for example, using the Glyco-Peakfinder, which is a piece of glycan search software freely available on an Internet website (see Non-Patent Literature 6). Needless to say, other software products may also be used to perform similar processing.

In estimating the monosaccharide composition, it is necessary to specify the kinds of monosaccharides and the range of the number of occurrences of each monosaccharide in a slightly broader way in order to avoid an omission of the correct monosaccharide composition. Therefore, two or more monosaccharide composition candidates will inevitably be listed for each peak. In the case of the already described example of Fig. 5, ten monosaccharide composition candidates are listed for a peak of m/z=3529.4. Accordingly, the next task is to narrow down the monosaccharide composition candidates for each peak, using the information on the mass difference corresponding to the technique used for the linkage-type-specific modification process performed in Step S2 (Step S5). The method for this narrowing process is hereinafter described with reference to Figs. 2 and 3.

Now, consider the case where the sialyl glycan has a "two-antenna" (two-branched) structure with one sialic acid linked to the terminal of each chain. In this case, as shown in boxes (a)-(c) in Fig. 2, there are three possible combinations of the α2,3-sialic acid and α2,6-sialic acid. The three combinations have the same mass and cannot be distinguished from each other by their masses. After being subjected to the linkage-type-specific modification as described earlier, the α2,3-sialic acid residue becomes the MA modification, while the α2,6-sialic acid residue becomes the iPA modification. Accordingly, there are three possible combinations of the modifications as shown in boxes (d)-(f) in Fig. 2. As noted earlier, the MA and iPA modifications have a mass difference of 28 Da. Therefore, the masses of the three combinations of the modifications differ from each other in units of 28 Da. That is to say, the sialyl glycans which have undergone the linkage-type-specific modification have distinguishable masses which depend on the linkage types of the sialic acid residues.

The three aforementioned combinations will appear as three peaks P1, P2 and P3 at intervals of 28 Da on a mass spectrum obtained by a mass spectrometric analysis of the sialylated glycans which have been modified in the linkage-type-specific way. Accordingly, if there are a plurality of peaks observed at intervals of 28 Da on a mass spectrum, those peaks are most likely to have originated from glycans which merely differ from each other in the kind of modification of the sialic acid residue, i.e. which are identical in the monosaccharide composition exclusive of the sialic acid residues as well as in the number of sialic acid residues. Such a group of peaks are assumed to be an "isomer cluster peak", i.e. a group of peaks which are identical in the monosaccharide composition exclusive of the sialic acid residues as well as in the number of sialic acid residues while being merely different from each other in the linkage type of the sialic acid residues.

The correspondence relationship between the three combinations of the modifications and the three peaks in Fig. 2 demonstrates the following facts:
(A1) The monosaccharide compositions corresponding to the three peaks must include a modified sialic acid residue.
(A2) The monosaccharide composition corresponding to the smaller-mass peak of any two peaks located at an interval of 28 Da includes one or more MA modifications, i.e. the α2,3-sialic acid residues.
(A3) The monosaccharide composition corresponding to the larger-mass peak of any two peaks located at an interval of 28 Da includes one or more iPA modifications, i.e. the α2,6-sialic acid residues.
(A4) For a glycan having two sialic acid residues, if three peaks are observed at intervals of 28 Da, the monosaccharide composition corresponding to the peak having the largest mass includes only the iPA modification, while the monosaccharide composition corresponding to the peak having the smallest mass includes only the MA modification.
(A5) The glycans are identical in the monosaccharide composition exclusive of the sialic acid residues.

Monosaccharide composition candidates which do not satisfy those conditions (A1) to (A5) can be excluded from the candidates. Thus, the narrowing of the candidates can be achieved.

Next, consider the case of a sialylated glycan having a "three-antenna" (three-branched) structure with one sialic acid linked to the terminal of each chain. In this case, as shown in boxes (a)-(d) in Fig. 3, there are four possible combinations of the α2,3-sialic acid and α2,6-sialic acid. The four combinations have the same mass and cannot be distinguished from each other by their masses. After the linkage-type-specific modification is performed as described earlier, there are four possible combinations of the modifications as shown in boxes (e)-(h) in Fig. 3. As noted earlier, the MA and iPA modifications have a mass difference of 28 Da. Therefore, the masses of the four combinations of the modifications differ from each other in units of 28 Da. That is to say, the sialyl glycans which have undergone the linkage-type-specific modification have distinguishable masses which depend on the linkage types of the sialic acid residues.

The four aforementioned combinations will appear as four peaks P1, P2, P3 and P4 at intervals of 28 Da on a mass spectrum obtained by a mass spectrometric analysis of the sialyl glycans which have been modified in the linkage-type-specific way. Accordingly, similar to the previous case, if there are a plurality of peaks observed at intervals of 28 Da on a mass spectrum, those peaks can be assumed to be an isomer cluster peak. Similar to the previous case in which the number of sialic acid residues is two, the following conditions can be applied to the isomer cluster peak.
(B1) The monosaccharide compositions corresponding to the four peaks must include a modified sialic acid residue.
(B2) The monosaccharide composition corresponding to the smaller-mass peak of any two peaks located at an interval of 28 Da includes one or more MA modifications, i.e. the α2,3-sialic acid residues.
(B3) The monosaccharide composition corresponding to the larger-mass peak of any two peaks located at an interval of 28 Da includes one or more iPA modifications, i.e. the α2,6-sialic acid residues.
(B4) The monosaccharide composition corresponding to a peak along with N peaks located at intervals of 28 Da on the larger-mass side includes N or more MA modifications, i.e. the α2,3-sialic acid residues.
(B5) The monosaccharide composition corresponding to a peak along with N peaks located at intervals of 28 Da on the smaller-mass side includes N or more iPA modification, i.e. the α2,6-sialic acid residues.

Monosaccharide composition candidates which do not satisfy those conditions (B1) to (B5) can be excluded from the candidates. Thus, the narrowing of the candidates can be achieved.

As described to this point, the monosaccharide composition candidates which are related to a plurality of peaks belonging to one isomer cluster peak identified by the mass difference of the peaks on a mass spectrum can be appropriately narrowed down by excluding candidates which are inconsistent with specific judgment conditions, such as the presence or absence of a modified sialic acid residue, presence or absence of a specific type of modified sialic acid residue, number of occurrences of the modification of the specific type of modified sialic acid residue, or identity of the monosaccharide composition exclusive of the sialic acid residues. Such a narrowing process can also be performed on a computer. The monosaccharide composition candidates which remain after the narrowing process are presented to the analysis operator as the analysis result, for example, on the screen of a display unit (Step S6).

### EXAMPLE

An experimental example in which the sialyl glycan analysis method according to the present embodiment was applied is hereinafter described in detail.

### [Step S1] Release and Purification of Glycans

In the experiment, a sample was prepared in the following manner by releasing glycans from a glycoprotein which includes both α2,3-linked and α2,6-linked sialyl glycan.

Initially, bovine fetuin was dissolved in a mixed liquid containing ammonium bicarbonate in a concentration of 20 mM, dithiothreitol (DTT) in a concentration of 10 mM and sodium dodecyl sulfate (SDS) in a concentration of 0.02 %. The solution was heated at 100°C for three minutes to denature and reduce the fetuin. Subsequently, the solution was cooled to room temperature. After PNGase F (Peptide-N-Glycosidase F) enzyme was added, the solution was incubated at 37°C overnight to release glycans from the peptides. Then, the solution was heated at 100°C for three minutes to deactivate PNGase F and thereby discontinue the enzymatic reaction.

The glycans released by the enzymatic reaction were subsequently desalted and purified using a carbon column. This carbon column was a self-build microchip similar to a purification column. It was prepared by filling a 200-µL column chip with a stack of active-carbon discs of approximately 1 mm in diameter punched from a carbon disk (Empore^{®} disk carbon). Initially, 100 µL of acetonitrile (ACN) was put into this carbon column, and the solution was centrifugally discharged. Similar operations were also performed using each of the following solutions: 1M sodium hydroxide (NaOH), 1M hydrochloric acid (HCl), 60% ACN, 0.1% trifluoroacetic acid (TFA) solution, and water, in order to wash and equilibrate the column adsorbent. The solution obtained through the enzymatic reaction was introduced into this column, and the solution was centrifugally discharged. An operation of adding 200 µL of water and centrifugally discharging the liquid was repeated three times for washing. Finally, an operation of adding 20 µL of a mixed liquid of 60% ACN and 0.1% TFA solution and centrifugally collecting the solution was repeated two times to elute the glycans. The entire eluate obtained through this two-time operation was put together, and the solvent was removed by a centrifugal vacuum concentrator to obtain the sample in a dried form.

### [Step S2] Linkage-type-specific Modification of Sialic Acid Residues

To the dried sample, 10 µL of a 4M solution of isopropylamine hydrochloride dissolved in dimethyl sulfoxide (DMSO) was added. Then, 10 µL of a solution prepared by dissolving diisopropyl carbodiimide (DIC) and 1-hydroxy benzotriazole (HOBt) in DMSO so that each solute was contained in a concentration of 500 mM was added as a dehydration condensation agent. The mixed solution was stirred at room temperature for two minutes and made to react with each other at 37°C for one hour. After the reaction, the solution was diluted with 120 µL of 93.3% ACN and 0.13% TFA solution. An excessive amount of reagent was removed by GL-Tip^{®} Amide, manufactured by GL Sciences Inc. This was specifically performed as follows: An operation of adding 100 µL of water to GL-Tip Amide and centrifugally discharging the solution was repeated three times to wash the tip. Next, an operation of adding 100 µL of 90% ACN and 0.1% TFA solution and centrifugally discharging the solution was repeated three times to equilibrate the tip. Then, the diluted solution after the reaction was entirely added and centrifugally processed to make the glycans be adsorbed onto the adsorbent. Subsequently, an operation of adding 200 µL of 90% ACN and 0.1% TFA solution and centrifugally discharging the solution was repeated three times to wash the tip. Finally, an operation of adding 10 µL of water and centrifugally discharging the water was repeated two times to elute the glycans. The entire eluate obtained through this two-time operation was put together, and the solvent was removed by a centrifugal vacuum concentrator to obtain the sample in a dried form.

Subsequently, 10 µL of a 2M solution of methylamine hydrochloride dissolved in DMSO was added to the dried sample. Then, 10 µL of a solution of tripyrrolizinophosphonium hexafluorophosphate (PyBOP) dissolved in 30% N-methylmorpholine (NMM) to a molar concentration of 500 mM was added as a dehydration condensation agent. The mixed solution was stirred at room temperature for one hour to promote the reaction. After the reaction, 120 µL of 93.3% ACN and 0.13% TFA solution was added to the solution. The previously described operations for the purification and elution using GL-Tip Amide were similarly performed. The collected eluate was processed by the centrifugal vacuum concentrator to obtain the sample in a dried form.

Through the previously described processes, a sample in which the sialic acids linked to the glycans are modified in a linkage-specific way can be prepared.

### [Step S3] Execution of Mass Spectrometric Analysis

The dried sample was re-dissolved in an appropriate amount of water. A fraction (1 µL) of the obtained solution was dropped on a MALDI focus plate, to which 0.5 µL of a 50% ACN solution containing 100 mM 3AQ/CA and 2 mM ammonium sulfate was added as the matrix. The plate was placed on a heat block at 75°C and left intact for 1.5 hours. This is to promote the reaction of labelling the reducing end of the glycans with 3AQ. After the reaction was completed, the plate was cooled to room temperature, and a mass spectrometric analysis in the negative-ion mode was performed using a MALDI-QIT-TOFMS (AXIMA Resonance, manufactured by Shimadzu Corporation and Kratos Analytical Ltd.) as the mass spectrometer, to obtain a mass spectrum over a predetermined range of mass-to-charge ratios. For the measurement, a technique called "On-Target 3AQ Labeling" was used, which is a high-sensitivity glycan detection method proposed by the applicant in Non-Patent Literature 7, 8 and other documents. The measurement technique is not limited to this one.

### [Step S4] Estimation of Monosaccharide Composition

For the estimation of the monosaccharide composition, Glyco-Peakfinder was used, which is a piece of software for searching for monosaccharide composition candidates which are consistent with the mass-to-charge-ratio values detected on a mass spectrum. This software basically does not use any database; it performs an exhaustive search for computing all possible combinations of the known masses of the monosaccharides and the number of occurrences of each monosaccharide. The aforementioned software in its original setting does not consider modifications of sialic acids. Therefore, the masses of the modified sialic acid residues were additionally set as optional monosaccharide residues for the search.

The kinds of monosaccharides and the range of the number of occurrences of each monosaccharide specified as the search conditions were as follows:
- Hexose (Hex): 3 to 15
- N-acetylhexosamine (HexNAc): 2 to 24
- Deoxyhexose (dHex): 0 to 4
- N-acetylneuraminic acid (Neu5Ac): 0 to 5 (or 0 to 5 for each mass if Neu5Ac in the modified form has a different mass depending on the linkage type)
- Sulfate (S): 0 to 2

The search result was as shown in Fig. 5, which has already been mentioned.

### [Step S5] Narrowing of Monosaccharide Composition Candidates

### <In the Case of Biantennary Glycan + Two Sialic Acid Residues>

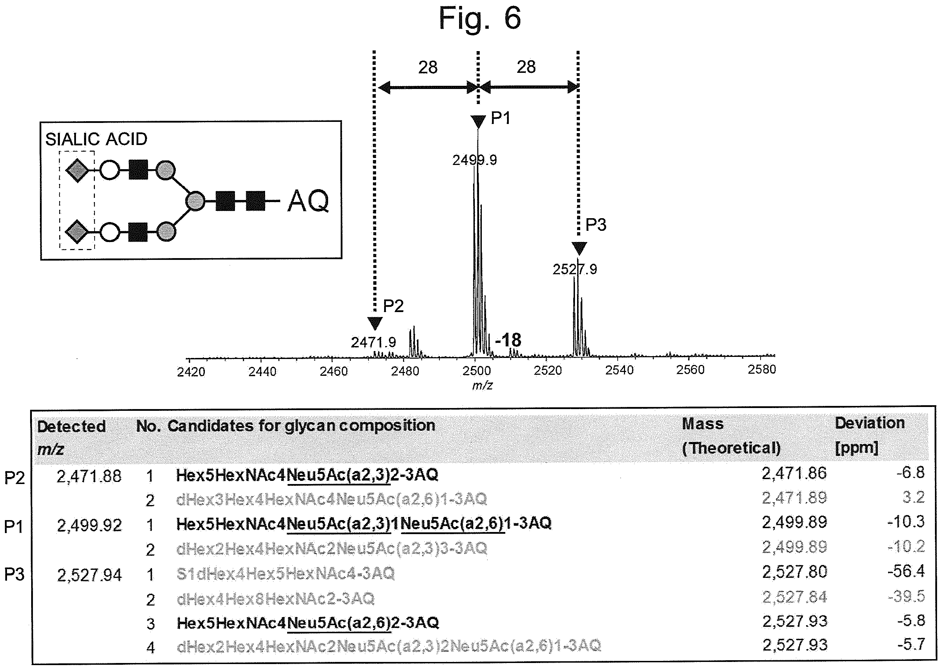

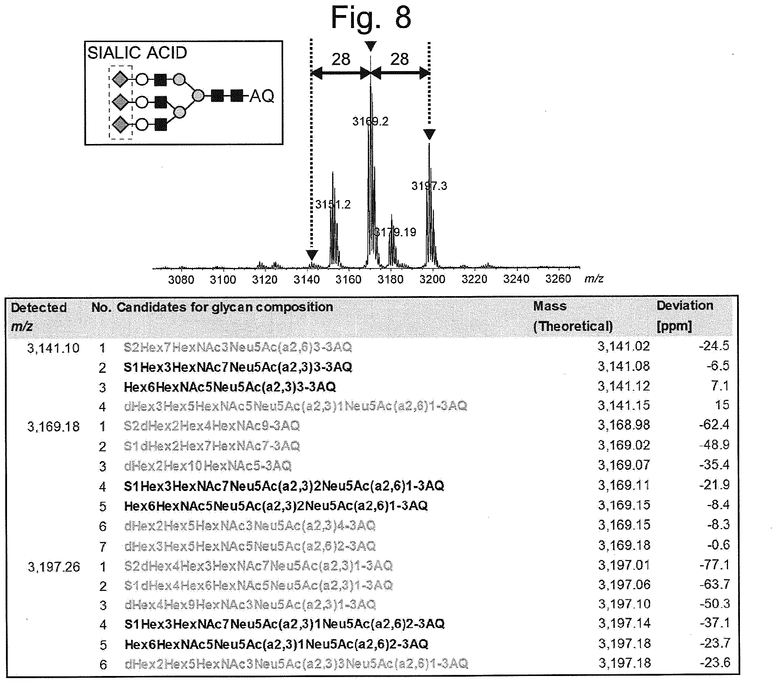

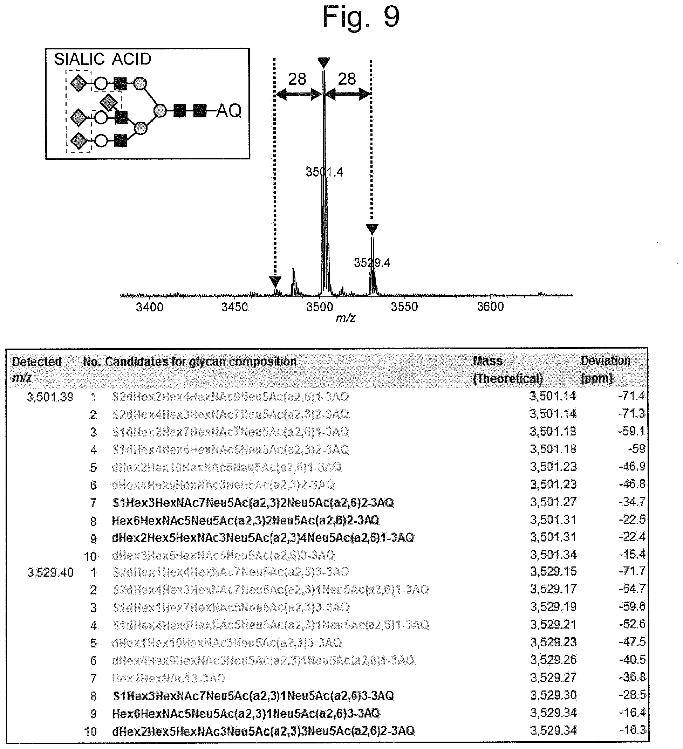

Fig. 6 shows an enlarged view of a section of the mass spectrum around m/z=2500 and a list showing the result of a monosaccharide composition search within the enlarged section. Candidates which have been excluded by the narrowing process are indicated in faint-colored letters in the list showing the result of the monosaccharide composition search in Fig. 6 (as well as in Figs. 8 and 9).

The peak observed at m/z=2471.9 in the mass spectra shown in Figs. 4 and 5 can be identified, from its mass-to-charge-ratio value, as a biantennary glycan with two sialic acid residues added, as with the glycan structure shown in Fig. 6. In the mass spectrum shown in Fig. 6, the peak of m/z=2499.9 is accompanied by two peaks on both sides, with each peak distanced from the central peak by 28 Da which is based on the difference in mass-to-charge ratio between the α2,3-sialic acid residue and the α2,6-sialic acid residue (i.e. between the MA and iPA modifications). These three peaks can be considered as forming an isomer cluster peak. As described earlier, a glycan which corresponds to three peaks belonging to an isomer cluster peak must include a sialic acid residue. Therefore, among the monosaccharide composition candidates related to those three peaks, those which include no sialic acid residue should be excluded from the candidates. In the example of Fig. 6, the first and second candidates among the four candidates related to peak P3 include no sialic acid residue and should be excluded.

The monosaccharide which corresponds to a peak accompanied by another peak with a mass difference of 28 Da on the larger-mass side on the mass spectrum among the three aforementioned peaks must have one or more MA modifications in its composition. Accordingly, among the monosaccharide composition candidates related to that peak, those which include no MA modification should be excluded from the candidates. In the example of Fig. 6, the second candidate among the two candidates related to peak P2 includes no α2,3-sialic acid residue and should be excluded.

Conversely, the monosaccharide which corresponds to a peak accompanied by another peak with a mass difference of 28 Da on the smaller-mass side on the mass spectrum must have one or more iPA modification in its composition. Accordingly, among the monosaccharide composition candidates related to that peak, those which include no iPA modification should be excluded from the candidates. In the example of Fig. 6, the second candidate among the two candidates related to peak P1 includes no a2,6-sialic acid residue and should be excluded.

Additionally, the monosaccharide corresponding to peak P3 must include two or more iPA modifications in its composition, since peak P2 is located at a position corresponding to a mass difference of 56 Da, which equals two times 28 Da, on the smaller-mass side from peak P3. Therefore, among the two remaining candidates related to peak P3, the fourth candidate which includes only one α2,6-sialic acid residue should be excluded.

The monosaccharide composition candidates can be narrowed down by such a process. In the example of Fig. 6, the monosaccharide composition candidates related to the three peaks can be narrowed down to a single candidate for each peak.

### <In the Case of Triantennary Glycan + Two Sialic Acid Residues>

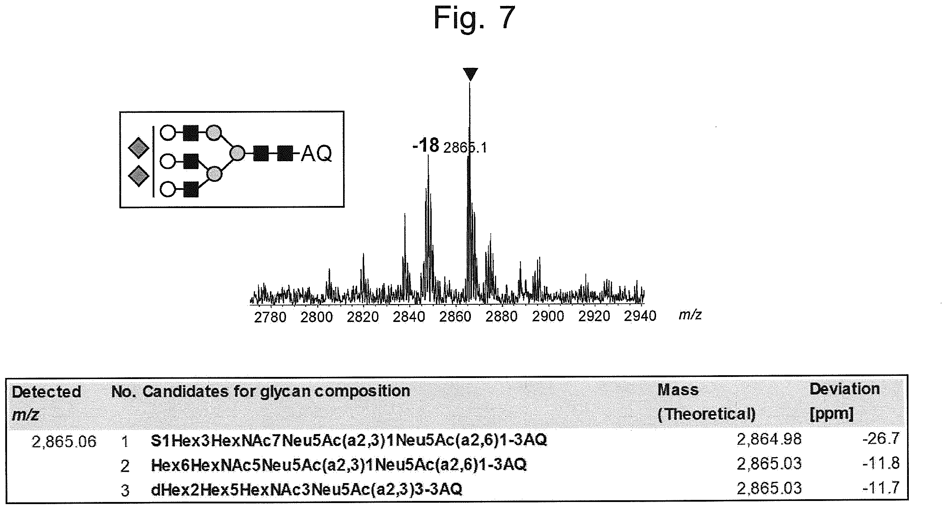

Fig. 7 shows an enlarged view of a section of the mass spectrum around m/z=2800 and a list showing the result of a monosaccharide composition search within the enlarged section. In the present case, there are three estimated monosaccharide compositions for the peak of m/z=2865.1. However, there is no isomer cluster peak observed with a significant intensity, i.e. with an intensity equal to or higher than a predetermined threshold. Therefore, in the present case, it is impossible to narrow down the candidates using an isomer cluster in the previously described manner.

### <In the Case of Triantennary Glycan + Three Sialic Acid Residues>

Fig. 8 shows an enlarged view of a section of the mass spectrum around m/z=3150 and a list showing the result of a monosaccharide composition search within the enlarged section.

The peak located at m/z=3141.1 can be identified, from its mass-to-charge-ratio value, as a triantennary glycan with three sialic acid residues added, as with the glycan structure shown in Fig. 8. There are four monosaccharide composition candidates listed for this peak of m/z=3141.1. There are two peaks observed at positions of +28 Da and +56 Da (=28×2) from this peak, respectively. Accordingly, it is possible to consider that these three peaks form an isomer cluster peak.

Among the monosaccharide composition candidates corresponding to the peak of m/z=3141.1 belonging to this isomer cluster peak, the first candidate which includes no α2,3-sialic acid residue (Neu5Ac(a2,3)) and the fourth candidate which includes only one α2,3-sialic acid residue can be excluded. The peak of m/z=3169.1 located at the center of the three peaks has seven monosaccharide composition candidates, among which the first through third candidates which include no sialic acid residue can be excluded. The sixth and seventh candidates can also be excluded, since any candidate for this peak should include at least one α2,3-sialic acid residue (Neu5Ac(a2,3)) and at least one α2,6-sialic acid residue (Neu5Ac(a2,6)). The peak located at the highest mass-to-charge ratio of m/z=3197.3 among the three peaks has six monosaccharide composition candidates, among which the first through third candidates as well as the sixth candidate can be excluded, since any candidate for this peak should include two or more α2,6-sialic acid residues (Neu5Ac(a2,6)).

Thus, the number of monosaccharide composition candidates can be decreased from 17 to 6 by assuming that the three peaks having a mass difference of 28 Da form an isomer cluster peak.

### <In the Case of Triantennary Glycan + Four Sialic Acid Residues>

Fig. 9 shows an enlarged view of a section of the mass spectrum around m/z=3500 and a list showing the result of a monosaccharide composition search within the enlarged section.

The peak located at m/z=3501.4 can be identified, from its mass-to-charge-ratio value, as a three-antenna glycan with four sialic acid residues added, as with the glycan structure shown in Fig. 9. Since there are four sialic acid residues, there are four possible combinations of the α2,3-sialic acid residue and the a2,6-sialic acid residue. However, only three peaks having a mass-to-charge-ratio difference of 28 Da will be observed on the mass spectrum. Even in this case, it is possible to exclude monosaccharide composition candidates based on similar rules to the previously described ones.

That is to say, for the peak of m/z=3501.4, a peak is observed at a position of +28 Da. Therefore, among the ten monosaccharide composition candidates corresponding to the peak of m/z=3501.4, the first, third, fifth and tenth candidates which include no α2,3-sialic acid residue should be excluded. Additionally, another peak is observed at a position of -28 Da, although its intensity is low. The presence of this peak allows for the exclusion of the second, fourth and sixth candidates which include no α2,6-sialic acid. Thus, the candidates can be narrowed down to three.

Similarly, among the ten monosaccharide composition candidates corresponding to the peak of m/z=3529.4, the seventh candidate which includes no sialic acid residue can be excluded. The presence of a peak at a position of -28 Da allows for the exclusion of the first, third and fifth candidates which include no α2,6-sialic acid residue. Furthermore, the presence of another peak at a position of -56 Da allows for the exclusion of the second, fourth and sixth candidates which include only one α2,6-sialic acid residue. Thus, the candidates can be narrowed down to three. Any of the monosaccharide composition candidates remaining after the narrowing process includes four sialic acid residues. In this manner, the candidates can be satisfactorily narrowed down even when all peaks that should belong to an isomer cluster peak are not completely observed.

In the narrowing method used in the previously described experiment, candidates are excluded based on the presence or absence of the sialic acid residue or its modification as well as the number of occurrences of the sialic acid residue or its modification. As described earlier, the narrowing of the candidates of a glycan corresponding to a peak belonging to an isomer cluster peak can also be achieved by excluding candidates which do no satisfy the condition that the monosaccharide composition exclusive of the modified sialic acid residues is the same, i.e. by excluding candidates having different monosaccharide compositions.

In the previously described embodiment, the isopropyl amide modification and the methyl amide modification are used as the linkage-type-specific modifications. A lactone modification may be used in place of the methyl amide modification. In that case, the two modifications have an nominal mass difference of 59 Da. Accordingly, an isomer cluster peak can be located by searching for peaks located at intervals of 59 Da or its integral multiple.

In the case of the linkage-type-specific modification method described in Non-Patent Literature 1 (lactone modification and methyl ester modification), the nominal mass difference between the two modifications is 32 Da. In the case of the linkage-type-specific modification method described in Non-Patent Literature 2 (lactone modification and ethyl ester modification), the nominal mass difference between the two modifications is 48 Da. In the case of the linkage-type-specific modification method described in Non-Patent Literature 3 (lactone modification and amide modification, followed by permethylation), the nominal mass difference between the two modifications is 13 Da. In the case of the linkage-type-specific modification method described in Non-Patent Literature 4 (lactone modification and dimethyl amide modification), the integer mass difference between the two modifications is 45 Da. In any of these cases, an isomer cluster peak can similarly be located by searching for peaks located at intervals of the mass difference or its integral multiple.

In the previously described embodiment, MALDI is used as the ionization method. It is naturally possible to apply the present invention in the case where a different ionization method is used, such as an electrospray ionization in which multiply-charged ions are generated. In the case where the sample is detected as a multiply-charged ion due to the use of such an ionization method, attention should be paid to the fact that the mass difference between the modifications to be considered must be divided by the number of the charge of the ion in order to locate a correct isomer cluster peak. Recent high-performance mass spectrometers can instantly determine the number of the charge of an ion based on the intervals of the isotopic masses calculated from an obtained mass spectrum. If such a mass spectrometer is used, the mass of a modified glycan can be automatically estimated from the determined charge. For the detection of an isomer cluster peak, the masses of the modified glycans estimated from mass-to-charge ratios may be used in place of the mass-to-charge ratios.

Although only the N-acetylneuraminic acid is considered as the sialic acid in the previously described embodiment, it is evident that the present invention is also applicable to other kinds of sialic acids, such as the N-glycolylneuraminic acid. It is also evident that the present invention is also applicable to any linkage type other than the α2,3- or α2,6-linkage as long as the sialic acid can be modified in a way that is specific to the linkage type concerned.

## Claims

1. A sialylated glycan analysis method using mass spectrometry for analyzing a structure of a sialylated glycan to which a sialic acid is linked, the method comprising:
a) a modification step in which a linkage-type-specific modification is performed on a sialic acid residue included in a sialylated glycan to be analyzed;
b) an analysis execution step in which the sialylated glycan after the modification by the modification step is subjected to a mass spectrometric analysis to obtain mass spectrum information;
c) a candidate estimation step in which a candidate of a monosaccharide composition is estimated for a peak observed on the mass spectrum obtained in the analysis execution step, based on mass information of the peak; and
d) a candidate-narrowing step in which a plurality of peaks observed on the mass spectrum and having a mass difference corresponding to a technique of the linkage-type-specific modification are assumed to be a cluster of peaks corresponding to sialylated glycans including sialic acid residues of a same kind with different linkage types and being identical in the monosaccharide composition exclusive of the sialic acid residues, and monosaccharide composition candidates obtained for those peaks are narrowed down by judging each monosaccharide composition candidate from at least one of following aspects:
(i) excluding a monosaccharide composition candidate which does not include a sialic acid residue;
(ii) excluding a monosaccharide composition candidate which does not include a specific type of modified sialic acid residue;
(iii) excluding a monosaccharide composition candidate which does not include a predetermined number of a specific type of modified sialic acid residue;
(iv) excluding a monosaccharide composition candidate which is not identical in a monosaccharide composition exclusive of the sialic acid residue.

2. The sialylated glycan analysis method according to claim 1, wherein:
an analysis of a glycan structure is performed in which α2,3-sialic acid residue and α2,6-sialic acid residue are distinguished from each other as the sialic acid residues with different linkage types.

3. The sialylated glycan analysis method according to claim 2, wherein:
an isopropylamide modification and a methylamide modification are performed as the linkage-type-specific modification in the modification step.

4. The sialylated glycan analysis method according to claim 1, wherein:
in the candidate-narrowing step, for a sialylated glycan having a sialic acid linked to one or more terminals of the glycan, a plurality of peaks corresponding to a mass difference between different types of modified sialic acid residue generated by the linkage-type-specific modification are located, and the monosaccharide composition candidates are narrowed down under a condition that the monosaccharide composition corresponding to a peak on a lower-mass side among the plurality of peaks should include at least one modified sialic acid residue having a smaller mass while the monosaccharide composition corresponding to a peak on a higher-mass side should include at least one modified sialic acid residue having a larger mass.

5. The sialylated glycan analysis method according to claim 4, wherein:
in the candidate-narrowing step, two peaks corresponding to N times the mass difference ΔM between the different types of modified sialic acid residue generated by the linkage-type-specific modification are located, and the monosaccharide composition candidates are narrowed down under a condition that the monosaccharide composition corresponding to the peak on the lower-mass side should include the modified sialic acid residue having the smaller mass and occurring at N locations, where N is an integer equal to or greater than one.

6. The sialylated glycan analysis method according to claim 5, wherein:
the modified sialic acid residue having the smaller mass and occurring at N locations is a modified α2,3-sialic acid residue.

7. The sialylated glycan analysis method according to claim 4, wherein:
in the candidate-narrowing step, two peaks corresponding to N times the mass difference ΔM between the different types of modified sialic acid residue generated by the linkage-type-specific modification are located, and the monosaccharide composition candidates are narrowed down under a condition that the monosaccharide composition corresponding to the peak on the higher-mass side should include the modified sialic acid residue having the larger mass and occurring at N locations, where N is an integer equal to or greater than one.

8. The sialylated glycan analysis method according to claim 7, wherein:
the modified sialic acid residue having the larger mass and occurring at N locations is a modified α2,6-sialic acid residue.

9. The sialylated glycan analysis method according to claim 1, wherein:
an estimation of a monosaccharide composition candidate in the candidate estimation step is performed by computing a combination of monosaccharides which are consistent with masses of the peaks under search conditions which specify kinds of potentially contained monosaccharides and a range of the number of occurrences of each monosaccharide.

10. The sialylated glycan analysis method according to claim 1, wherein:
the mass spectrometric analysis in the analysis execution step performed in a negative-ion mode.

## Patentansprüche

1. Verfahren zur Analyse von sialyliertem Glykan unter Verwendung von Massenspektrometrie zum Analysieren der Struktur eines sialylierten Glykans, an das Sialinsäure gebunden ist, wobei das Verfahren Folgendes umfasst:
a) einen Modifikationsschritt, bei dem eine bindungstypspezifische Modifikation an einem Sialinsäurerest durchgeführt wird, der in einem zu analysierenden sialylierten Glykan enthalten ist;
b) einen Analyse-Ausführungsschritt, bei dem das sialylierte Glykan nach der Modifikation durch den Modifikationsschritt einer massenspektrometrischen Analyse unterzogen wird, um Massenspektrum-Informationen zu erhalten;
c) einen Kandidaten-Bewertungsschritt, bei dem ein Kandidat für eine Monosaccharid-Zusammensetzung anhand eines Peaks, der in dem im Analyse-Ausführungsschritt erhaltenen Massenspektrum beobachtet wird, basierend auf den Masseninformationen des Peaks bewertet wird; und
d) ein Kandidaten-Eingrenzungsschritt, bei dem eine Vielzahl von Peaks, die in dem Massenspektrum beobachtet werden und einen Massenunterschied aufweisen, der einem Verfahren der bindungstypspezifischen Modifikation entspricht, als Cluster von Peaks angenommen werden, die sialylierten Glykanen entsprechen, die Sialinsäurereste der gleichen Art mit unterschiedlichen Bindungstypen umfassen und in der Monosaccharid-Zusammensetzung ausschließlich der Sialinsäurereste identisch sind, und für diese Peaks erhaltene Monosaccharid-Zusammensetzungskandidaten eingegrenzt werden, indem jeder Monosaccharid-Zusammensetzungskandidat nach zumindest einem der folgenden Aspekte beurteilt wird:
(i) Ausschließen eines Monosaccharid-Zusammensetzungskandidaten, der keinen Sialinsäurerest umfasst;
(ii) Ausschließen eines Monosaccharid-Zusammensetzungskandidaten, der keinen spezifischen Typ von modifizierten Sialinsäureresten umfasst;
(iii) Ausschließen eines Monosaccharid-Zusammensetzungskandidaten, der keine vorbestimmten Anzahl eines spezifischen Typs von modifiziertem Sialinsäurerest umfasst;
(iv) Ausschließen eines Monosaccharid-Zusammensetzungskandidaten, der in einer Monosaccharid-Zusammensetzung ausschließlich des Sialinsäurerests nicht identisch ist.

2. Verfahren zur Analyse von sialyliertem Glykan nach Anspruch 1, wobei:
eine Analyse der Glykanstruktur durchgeführt wird, in der ein α2,3-Sialinsäurerest und ein α2,6-Sialinsäurerest als Sialinsäurereste mit unterschiedlichen Bindungstypen voneinander unterschieden werden.

3. Verfahren zur Analyse von sialyliertem Glykan nach Anspruch 2, wobei:
im Modifikationsschritt als bindungstypspezifische Modifikation eine Isopropylamid-Modifikation und eine Methylamid-Modifikation durchgeführt werden.

4. Verfahren zur Analyse von sialyliertem Glykan nach Anspruch 1, wobei:
im Kandidateneingrenzungsschritt eine Vielzahl von Peaks, die einem Massenunterschied zwischen unterschiedlichen Typen von modifizierten Sialinsäureresten entsprechen, die durch die bindungsspezifische Modifikation erzeugt wurden, für ein sialyliertes Glykan, bei dem eine Sialinsäure an eine oder mehrere Endgruppen des Glykans gebunden ist, verortet werden und die Monosaccharid-Zusammensetzungskandidaten unter der Bedingung eingegrenzt werden, dass eine Monosaccharid-Zusammensetzung, die einem Peak mit geringerer Masse aus der Vielzahl von Peaks entspricht, zumindest einen modifizierten Sialinsäurerest mit einer geringeren Masse umfassen soll, während eine Monosaccharid-Zusammensetzung, die einem Peak mit größerer Masse entspricht, zumindest einen modifizierten Sialinsäurerest mit größerer Masse umfassen soll.

5. Verfahren zur Analyse von sialyliertem Glykan nach Anspruch 4, wobei:
im Kandidaten-Eingrenzungsschritt zwei Peaks, die N-mal dem Massenunterschied ΔM zwischen den unterschiedlichen Typen von modifizierten Sialinsäureresten entsprechen, die durch die bindungstypspezifische Modifikation erzeugt wurden, verortet werden und die Monosaccharid-Zusammensetzungskandidaten unter der Bedingung eingegrenzt werden, dass die Monosaccharid-Zusammensetzung, die dem Peak mit geringerer Masse entspricht, den modifizierten Sialinsäurerest mit geringerer Masse, der an N Stellen vorkommt, umfassen soll wobei N eine ganze Zahl größer oder gleich eins ist.

6. Verfahren zur Analyse von sialyliertem Glykan nach Anspruch 5, wobei:
der modifizierte Sialinsäurerest, der die geringere Masse aufweist und an N Stellen vorkommt, ein modifizierter a2,3-Sialinsäurerest ist.

7. Verfahren zur Analyse von sialyliertem Glykan nach Anspruch 4, wobei:
im Kandidateneingrenzungsschritt zwei Peaks, die N-mal dem Massenunterschied ΔM zwischen den unterschiedlichen Typen von modifizierten Sialinsäureresten entsprechen, die durch die bindungstypspezifische Modifikation erzeugt wurden, verortet werden und die Monosaccharid-Zusammensetzungskandidaten unter der Bedingung eingegrenzt werden, dass die Monosaccharid-Zusammensetzung, die dem Peak mit größerer Masse entspricht, den modifizierten Sialinsäurerest mit größerer Masse, der an N Stellen vorkommt, umfassen soll, wobei N eine ganze Zahl größer oder gleich eins ist.

8. Verfahren zur Analyse von sialyliertem Glykan nach Anspruch 7, wobei:
der modifizierte Sialinsäurerest, der die größere Masse aufweist und an N Stellen vorkommt, ein modifizierter α2,6-Sialinsäurerest ist.

9. Verfahren zur Analyse von sialyliertem Glykan nach Anspruch 1, wobei:
die Bewertung eines Monosaccharid-Zusammensetzungskandidaten im Kandidaten-Bewertungsschritt durch Berechnen einer Kombination aus Monosacchariden durchgeführt wird, bei denen die Peakmassen unter Suchbedingungen, bei denen Arten von potentiell enthaltenen Monosacchariden und ein Bereich der Anzahl des Vorkommens jedes Monosaccharids spezifiziert werden, übereinstimmen.

10. Verfahren zur Analyse von sialyliertem Glykan nach Anspruch 1, wobei:
die massenspektrometrische Analyse im Analyse-Ausführungsschritt im Negativionen-Modus durchgeführt wird.

## Revendications

1. Procédé d'analyse de glycane sialylé utilisant la spectrométrie de masse pour analyser une structure d'un glycane sialylé auquel un acide sialique est lié, le procédé comprenant :
a) une étape de modification dans laquelle une modification spécifique de type de liaison est effectuée sur un résidu d'acide sialique inclus dans un glycane sialylé devant être analysé ;
b) une étape d'exécution d'analyse dans laquelle le glycane sialylé, après la modification par l'étape de modification, est soumis à une analyse spectrométrique de masse pour obtenir des informations de spectre de masse ;
c) une étape d'estimation de candidat dans laquelle un candidat d'une composition monosaccharidique est estimé pour un pic observé sur le spectre de masse obtenu dans l'étape d'exécution d'analyse, sur la base d'informations de masse du pic ; et
d) une étape de restriction de candidats dans laquelle une pluralité de pics observés sur le spectre de masse et présentant une différence de masse correspondant à une technique de la modification spécifique de type liaison sont supposés être un groupe de pics correspondant à des glycanes sialylés comprenant des résidus d'acide sialique d'un même type avec différents types de liaison et étant identiques dans la composition de monosaccharide à l'exclusion des résidus d'acide sialique, et les candidats de composition monosaccharidique obtenus pour ces pics sont restreints en jugeant chaque composition monosaccharidique candidate par rapport à au moins un des aspects suivants :
i) exclusion d'un candidat de composition monosaccharidique qui ne comprend pas de résidu d'acide sialique ;
ii) exclusion d'un candidat de composition monosaccharidique qui ne comprend pas un type spécifique de résidu d'acide sialique modifié ;
iii) exclusion d'un candidat de composition monosaccharidique qui ne comprend pas un nombre prédéterminé d'un type spécifique de résidu d'acide sialique modifié ;
iv) exclusion d'un candidat de composition monosaccharidique qui n'est pas identique dans une composition monosaccharidique à l'exclusion du résidu d'acide sialique.

2. Procédé d'analyse de glycane sialylé selon la revendication 1, dans lequel :
une analyse d'une structure de glycane est effectuée dans laquelle un résidu d'acide α2,3-sialique et un résidu d'acide α2,6-sialique sont distingués l'un de l'autre en tant que résidus d'acide sialique avec différents types de liaison.

3. Procédé d'analyse de glycane sialylé selon la revendication 2, dans lequel :
une modification d'isopropylamide et une modification de méthylamide sont effectuées en tant que modification spécifique de type de liaison dans l'étape de modification.

4. Procédé d'analyse de glycane sialylé selon la revendication 1, dans lequel :
dans l'étape de restriction de candidats, pour un glycane sialylé présentant un acide sialique lié à une ou plusieurs terminaisons du glycane, une pluralité de pics correspondant à une différence de masse entre différents types de résidu d'acide sialique modifié générés par la modification spécifique de type liaison sont localisés, et les candidats de composition monosaccharidique sont restreints à condition que la composition monosaccharidique correspondant à un pic sur un côté de masse inférieure parmi la pluralité de pics comprenne au moins un résidu d'acide sialique modifié présentant une masse plus petite tandis que la composition monosaccharidique correspondant à un pic sur un côté de masse supérieure comprenne au moins un résidu d'acide sialique modifié présentant une masse plus grande.

5. Procédé d'analyse de glycane sialylé selon la revendication 4, dans lequel :
dans l'étape de restriction de candidats, deux pics correspondant à N fois la différence de masse ΔM entre les différents types de résidu d'acide sialique modifié générés par la modification spécifique de type liaison sont localisés, et les candidats de composition de monosaccharide sont restreints à condition que la composition monosaccharidique correspondant au pic sur le côté de masse inférieure comprenne le résidu d'acide sialique modifié présentant la masse inférieure et survenant à N emplacements, où N est un entier égal ou supérieur à un.

6. Procédé d'analyse de glycane sialylé selon la revendication 5, dans lequel :
le résidu d'acide sialique modifié présentant la masse plus petite et survenant à N emplacements est un résidu d'acide α2,3-sialique modifié.

7. Procédé d'analyse de glycane sialylé selon la revendication 4, dans lequel :
dans l'étape de restriction de candidats, deux pics correspondant à N fois la différence de masse ΔM entre les différents types de résidu d'acide sialique modifié générés par la modification spécifique de type liaison sont localisés, et les candidats de composition de monosaccharide sont restreints à condition que la composition monosaccharidique correspondant au pic sur le côté de masse supérieure comprenne le résidu d'acide sialique modifié présentant la masse supérieure et survenant à N emplacements, où N est un entier égal ou supérieur à un.

8. Procédé d'analyse de glycane sialylé selon la revendication 7, dans lequel :
le résidu d'acide sialique modifié présentant la masse supérieure et survenant à N emplacements est un résidu d'acide α2,6-sialique modifié.

9. Procédé d'analyse de glycane sialylé selon la revendication 1, dans lequel :
une estimation d'une composition monosaccharidique candidate dans l'étape d'estimation de candidat est effectuée par calcul d'une combinaison de monosaccharides qui sont cohérents avec des masses des pics dans des conditions de recherche qui spécifient des types de monosaccharides potentiellement contenus et une plage du nombre d'occurrences de chaque monosaccharide.

10. Procédé d'analyse de glycane sialylé selon la revendication 1, dans lequel :
l'analyse spectrométrique de masse dans l'étape d'exécution d'analyse est effectuée dans un mode d'ions négatifs.
